# DEMANDE DE BREVET EUROPEEN

(11) **EP 3 711 708 A1**
(43) Date de publication de la demande: **23.09.2020**
(21) Numéro de dépôt: 20164002.6
(22) Date de dépôt: 18.03.2020
(51) Int. Cl.: A61F 2/00, A61B 17/04, A61B 17/00, A61B 17/06

(54) **DISPOSITIF COMBINANT SUSPENSION ET MISE EN TENSION RÉAJUSTABLE, RÉVERSIBLE ET AMOVIBLE POUR LE TRAITEMENT DE L'INCONTINENCE URINAIRE**

(30) Priorité: 18.03.2019 FR 1902774
(71) Demandeur: Devonec, Marian, 01700 Miribel (FR)
(72) Inventeur: Devonec, Marian, 01700 Miribel (FR)
(74) Mandataire: Chevalier, Renaud Philippe

(57) **Abrégé**

L'invention concerne un dispositif médical pour le traitement de l'incontinence urinaire comprenant :
- au moins un fil de suspension (21) présentant une première extrémité comportant un élément de suspension et une seconde extrémité libre,
- au moins un moyen de mise en tension (30) d'un fil de suspension comprenant un élément de passage et d'appui (31) pour la seconde extrémité dudit fil de suspension pour un élément de blocage (40) de la tension du fil de suspension, ledit élément de passage et d'appui présentant au moins un chenal (32) avec un orifice d'entrée (32a) et un orifice de sortie (32b), et ledit élément de blocage de la tension du fil de suspension étant conçu pour bloquer de manière amovible et réversible la seconde extrémité du fil de suspension au-delà de l'orifice de sortie dudit élément de passage et d'appui.

## Description

L'incontinence urinaire est la perte d'urine involontaire conséquence de l'affaiblissement ou de la perte du contrôle du sphincter urinaire. Il existe plusieurs types d'incontinence urinaire, la plus fréquente étant l'incontinence urinaire d'effort. La fuite d'urine s'observe lorsque la pression exercée sur la vessie est supérieure à la pression de fermeture du sphincter strié de l'urètre. L'augmentation de la pression exercée sur la vessie s'observe lors de la toux, l'éternuement, le rire, l'exercice physique et le port de charge lourde. Chez la femme, l'incontinence urinaire est la conséquence d'une variété de facteurs tels que l'affaiblissement musculaire après accouchement, la ménopause, le relâchement musculaire lié à l'âge. L'association de ces facteurs aboutit à une hypermobilité du plancher pelvien et de l'urètre. Alors que l'incontinence urinaire d'effort est plus fréquemment observée chez la femme, l'homme peut également en souffrir. C'est la raison pour laquelle une solution doit pouvoir être appliquée à la fois à l'anatomie féminine et à l'anatomie masculine. Chez l'homme la chirurgie du cancer de la prostate est une des causes principales de l'incontinence d'effort, conséquence d'une atteinte directe du sphincter strié de l'urètre et/ou indirectement de son innervation.

Chez tout individu normal le stockage de l'urine se fait dans la vessie, située dans la partie basse de l'abdomen, c'est à dire dans le pelvis. La vessie se vidange par le canal de l'urètre, un tube par lequel l'urine est éliminée du corps. Pendant la miction, le muscle sphinctérien entourant l'urètre se relâche volontairement et permet l'élimination de l'urine de la vessie. Chez un individu souffrant d'incontinence urinaire, le muscle sphinctérien est en permanence en situation affaiblie ou relâchée. Ceci permet à l'urine de s'écouler involontairement le long de l'urètre. Ceci explique les fuites répétées d'urine et leur prévention par des mictions fréquentes. L'insuffisance sphinctérienne est souvent minime. Le traitement chirurgical corrige le relâchement de la musculature du patient. La récidive des fuites urinaires après traitement chirurgical est fréquente du fait du relâchement continu de la musculature du patient avec la progression de son âge. Il n'est pas possible de retendre le matériel laissé en place lors de la première opération. Une nouvelle opération est nécessaire. La deuxième opération, lorsqu'elle est indiquée et réalisée consiste à reproduire la première technique avec un matériel de même nature ou à utiliser une autre technique avec un matériel différent.

Le traitement de l'incontinence urinaire d'effort chez la femme peut être réalisé selon plusieurs techniques. La technique la plus fréquemment pratiquée utilise une bandelette synthétique tressée avec un fil formant des mailles et avec des pores entre les mailles. La bandelette est placée sous l'urètre par voie trans-vaginale. Selon la technique utilisée les deux extrémités de la bandelette sortent soit verticalement par voie rétro-pubienne à travers les muscles grands droits de l'abdomen, c'est la technique de la bandelette sans tension TVT décrite par Ulmsten, soit les deux extrémités sortent horizontalement par voie trans-obturatrice (TOT) à travers le trou obturateur de chaque côté du bassin, c'est la technique décrite par Delorme. La bandelette réalise une fronde passant au-dessous de l'urètre. Cette fronde est fermée sous la forme de la lettre U dans la technique par voie rétro-pubienne. La fronde est ouverte sous la forme d'une lettre V ouverte dans la technique trans-obturatrice. Ces techniques peu invasives ont progressivement remplacé les techniques classiques dont celle décrite par Burch et celle décrite par Goebel-Stockel. Ces deux techniques nécessitent une large incision sus-pubienne de façon à permettre un abord de la région péri-urétrale située au fond de la cavité pelvienne au contact du plancher musculo-aponévrotique pelvien. La technique de Burch consiste à créer un hamac vaginal soutenant l'urètre par des points de fils non résorbables passés de part et d'autre de l'urètre. Ces fils sont arrimés au ligament de Cooper situé de chaque côté de la ligne médiane sur la face interne de l'os du pubis. Les fils sont ensuite noués avec une certaine tension. La technique de Goebel-Stockel consiste à prélever une bandelette de fascia d'un muscle grand droit et à la faire passer en dessous de l'urètre. Chaque extrémité de la bandelette est fixée par des points de fil non résorbable au ligament de Cooper situé sur le versant interne de chaque côté de l'os du pubis. La bandelette naturelle de fascia réalise une fronde autour de l'urètre. Elle est positionnée avec une certaine tension. Les inconvénients de ces techniques classiques sont leur caractère chirurgical invasif et la difficulté de juger du degré de tension des fils suspendant le hamac vaginal naturel ou du degré de tension donnée à la fonde de fascia naturel dans respectivement la technique de Burch et la technique de Goebel-Stockel. L'opérateur ne dispose d'aucun contrôle visuel à partir du vagin pour juger de l'effet de la tension sur la position du vagin et de l'urètre. Le résultat immédiat de ces techniques est souvent une tension trop importante du moyen de suspension et en conséquence une compression trop élevée de l'urètre avec une difficulté à la miction pour le patient et une rétention aiguë ou chronique d'urine. Aucun réajustement n'est possible une fois l'opération terminée.

Les bandelettes synthétiques selon la technique de TVT ou TOT ont l'avantage d'être peu invasives avec des incisions cutanées plus petites mais ont comme inconvénient de ne pas pouvoir être réajustée une fois l'opération terminée. En effet en 48 heures les tissus en contact colonisent les pores de la bandelette qui est irréversiblement incorporée et fixée dans les tissus. Ceci est un inconvénient, puisqu'avec le temps et la progression de l'âge du patient, le tonus musculaire du patient diminue et les fuites réapparaissent. La solution est alors une nouvelle intervention avec la pose d'une nouvelle bandelette. Les bandelettes ont en plus comme inconvénient d'être un corps étranger responsable d'érosion de l'urètre, de la vessie ou d'un cul de sac vaginal. Le contact de la bandelette avec l'urine ou avec la cavité vaginale est responsable d'infection sur le trajet de la bandelette. La guérison de l'infection n'est assurée que par l'ablation complète de l'ensemble du corps étranger. Ceci n'est réalisé que par une opération délabrante responsable de blessure d'autres organes avec des séquelles fonctionnelles neurologiques sévères parmi lesquelles une incontinence urinaire sévère. Le contact de la bandelette avec la vessie est responsable d'irritation neurologique du muscle vésical et d'hyperactivité de la vessie. Le contact avec le nerf obturateur est responsable de douleur dans la racine de la cuisse ou de paralysie des muscles adducteurs de la cuisse.

Pour diminuer les risques décrits plus haut, d'autres dispositifs ont été proposés avec une réduction de la longueur de la bandelette et permettre sa mise en place par une seule incision vaginale. La mini-bandelette comme la bandelette TVT ou TOT ne peut pas être réajustée après sa pose. Elle reste difficile à extraire en cas de complication d'érosion ou d'infection.

Plus récemment le dispositif Remeex (Neomedic International) a été proposé pour permettre le réajustement de la tension de la bandelette sous-urétrale. Le dispositif consiste en une boite implantable contenant un tambour autour duquel les extrémités libres des fils suspendant la bandelette sont enroulées. Le tambour comprend une roue dentée (brevet US publication 2008/0021263 A1). La boite est implantée dans la région sus-pubienne. Le réajustement nécessite une nouvelle incision en regard de la boite, la libération au bistouri électrique des tissus environnants qui ont colonisé la boite. Après libération des tissus, le réajustement est réalisé avec un tournevis chez une patient sous anesthésie. Le réajustement est réalisé dans des conditions éloignées des conditions naturelles chez une patiente sans anesthésie ou une simple anesthésie locale.

Wijay (Brevet US, US 8,211,004) a amélioré le design du dispositif original du Remeex. Le tambour est protégé de façon étanche par un emballage hermétique. Ceci prévient la colonisation par les tissus environnants cicatriciels. La fronde ne comprend pas de bandelette. La fronde est un fil qui passe dans un tube creux positionné en dessous et de chaque côté de l'urètre. Les deux extrémités libres du fil fronde sont enroulées autour du tambour identique à celui du dispositif Remeex comme décrit plus haut. La rotation du tambour dans le sens horaire ou anti-horaire avec un tournevis permet respectivement de tendre ou de détendre le fil.

Chez l'homme la technique de la bandelette par voie trans-obturatrice est utilisée pour le traitement de l'incontinence urinaire observée au cours des mois suivants la chirurgie pour cancer de la prostate par prostatectomie radicale. Une bandelette (par exemple Advance, AMS) est posée après une incision périnéale. Elle est passée entre les deux trous obturateurs et vient en contact avec l'urètre bulbaire. Elle est tendue au maximum de façon à soutenir l'urètre et le comprimer. Une fois posée elle n'est pas réajustable, puisqu'elle est colonisée par les tissus.

L'objectif du dispositif décrit ci-dessous est de remédier aux inconvénients des matériels et techniques décrits ci-dessus. Le matériel du dispositif proposé supprime la bandelette, il minimise le volume du matériel implanté, il est réajustable de façon réversible, il est amovible facilement et totalement si nécessaire.

Le dispositif médical pour le traitement de l'incontinence urinaire selon l'invention comprend :
- au moins un fil de suspension présentant une première extrémité comportant un élément de suspension et une seconde extrémité libre,
- au moins un moyen de mise en tension d'un fil de suspension comprenant un élément de passage et d'appui pour la seconde extrémité dudit fil de suspension pour un élément de blocage de la tension du fil de suspension, ledit élément de passage et d'appui présentant au moins un chenal avec un orifice d'entrée et un orifice de sortie, et ledit élément de blocage de la tension du fil de suspension étant conçu pour bloquer de manière amovible et réversible la seconde extrémité du fil de suspension au-delà de l'orifice de sortie dudit élément de passage et d'appui.

Selon l'invention, le fil de suspension peut être biocompatible, monobrin, non résorbable, mobilisable, amovible, non métallique ou métallique, de surface lisse ou avec crans ou ardillons disposés le long de la surface du fil et l'élément de suspension à l'extrémité opposée à l'extrémité libre dudit fil peut être une bille, un disque de surface lisse en matériau biocompatible métallique ou non métallique tel que titanium, verre, céramique, polypropylène ou autre composant synthétique

Selon l'invention, l'élément de passage du moyen de mise en tension peut être biocompatible, non résorbable, mobilisable, amovible, métallique ou non métallique, peut être une bille, un cylindre ou un disque perforé(e) avec un chenal dont le diamètre est adapté au diamètre du fil et du moyen de blocage, de façon à garantir à l'élément de blocage du moyen de mise en tension un appui ou accroche efficace sur l'élément de passage.

Selon l'invention, l'élément de blocage du moyen de mise en tension peut être biocompatible, non résorbable, mobilisable, métallique ou non métallique, amovible et repositionnable.

L'invention concerne également une méthode pour le traitement de l'incontinence urinaire comprenant :
- le passage de l'extrémité libre d'un fil de chaque côté de l'urètre à partir d'une incision vaginale, puis dans l'espace rétro-pubien et sortie par une incision dans la région sus-pubienne, l'extrémité opposée du fil comportant l'élément de suspension restant à distance du plan musculo-aponévrotique pelvien,
- le passage de l'extrémité libre de chacun des deux fils dans le chenal d'un élément de passage, et d'appui
- la mise en tension des deux fils jusqu'au contact de l'élément de suspension de chaque fil avec le plan musculo-aponévrotique pelvien.
le blocage de la position en tension des deux fils par un élément de blocage prenant appui sur l'élément de passage et d'appui du moyen de mise en tension, de façon à empêcher tout glissement des fils à l'intérieur du chenal de l'élément de passage et d'appui et la perte de la tension des fils.

L'invention sera décrite plus en détails à travers les différentes figures présentées ci-dessous, ce qui facilitera sa compréhension.
- La figure 1 est une vue frontale d'un premier moyen de suspension selon l'invention ;
- La figure 2 est une vue frontale d'un deuxième moyen de suspension selon l'invention ;
- La figure 3 est une vue frontale d'un troisième moyen de suspension selon l'invention ;
- La figure 4 est une vue frontale d'un quatrième moyen de suspension selon l'invention ;
- La figure 5 est une vue en perspective d'un premier élément de passage et d'appui selon l'invention ;
- La figure 6 est une vue en coupe frontale du premier élément de passage et d'appui selon l'invention ;
- La figure 7 est une vue en perspective d'un deuxième élément de passage et d'appui selon l'invention ;
- La figure 8 est une vue en coupe frontale du deuxième élément de passage et d'appui selon l'invention ;
- La figure 9 est une vue en perspective d'un troisième élément de passage et d'appui selon l'invention ;
- La figure10 est une vue en coupe frontale du troisième élément de passage et d'appui selon l'invention ;
- La figure 11 est une vue en perspective d'un quatrième élément de passage et d'appui selon l'invention ;
- La figure 12 est une vue en coupe frontale du quatrième élément de passage et d'appui selon l'invention ;
- La figure 13 est une vue en perspective d'un premier élément de blocage selon l'invention ;
- La figure 14 est une vue en coupe transversale du premier élément de blocage selon l'invention ;
- La figure 15 est une vue en perspective d'un deuxième élément de blocage selon l'invention ;
- La figure 16 est une vue en coupe transversale du deuxième élément de blocage selon l'invention ;
- La figure 17 est une vue en perspective d'un troisième élément de blocage selon l'invention ;
- La figure 18 est une vue en coupe transversale du troisième élément de blocage selon l'invention ;
- La figure 19 est une vue en perspective d'un quatrième élément de blocage selon l'invention ;
- La figure 20 est une vue en coupe transversale du quatrième élément de blocage selon l'invention ;
- La figure 21 est une vue en perspective d'un moyen de mise en tension selon un autre mode de réalisation selon l'invention ;
- La figure 22 est une vue en coupe frontale de la première étape du moyen de mise en tension représenté à la figure 21 ;
- La figure 23 est une vue en coupe frontale de la deuxième étape du moyen de mise en tension représenté à la figure 21 ;
- La figure 24 est une vue en coupe frontale de la troisième étape du moyen de mise en tension représenté à la figure 21 ;
- La figure 25 est une vue frontale de la première étape d'un exemple de méthode de pose d'un dispositif médical implantable selon un des modes de réalisation de l'invention ;
- La figure 26 est une vue frontale de la deuxième étape du mode de réalisation de l'invention représenté à la figure 25 ;
- La figure 27 est une vue frontale de la troisième étape du mode de réalisation de l'invention représenté à la figure 25 ;
- La figure 28 est une vue frontale après réajustement du moyen de suspension et du moyen de mise en tension du mode de réalisation de l'invention représenté à la figure 25 ;

Le sujet de la présente invention est celui des dispositifs médicaux implantables pour le traitement de l'incontinence urinaire. Le dispositif implantable proposé a comme caractéristique de pouvoir être utilisé préférentiellement chez la femme mais également chez l'homme. Le dispositif de traitement de l'incontinence urinaire a comme autre caractéristique d'être réajustable de façon réversible après implantation et amovible. Le dispositif comprend la coopération d'un moyen de suspension et d'un moyen de mise en tension. Le dispositif permet pendant l'effort le maintien de la position de l'urètre telle qu'elle est au repos.
- ledit moyen de suspension est au moins un fil dont une extrémité inférieure comprend un élément de suspension, l'autre extrémité supérieure étant libre, ledit élément de suspension est par exemple une bille ou une rondelle associée ou non à une rondelle grillagée perforée en son centre.
- ledit moyen de mise en tension du fil associe un élément de passage pour ledit fil et d'appui pour un élément de blocage de la tension du fil.
- ledit élément de passage dudit fil est par exemple une bille, un cylindre, ou un disque perforé(e) par un chenal avec un orifice d'entrée et un orifice de sortie.
- ledit élément de blocage de la tension du fil prend appui sur ledit élément de passage du fil et est indépendant ou intégré audit fil et caractérisé lorsqu'il est indépendant par un clip de blocage de la tension du fil ou lorsqu'il est intégré au fil par une série de nœuds entre deux fils ou par des crans ou ardillons disposés le long du fil.

La description détaillée du moyen de suspension comprenant un fil et un élément de suspension et du moyen de mise en tension comprenant un élément de passage et d'appui et un élément de blocage est présentée ci-dessous.

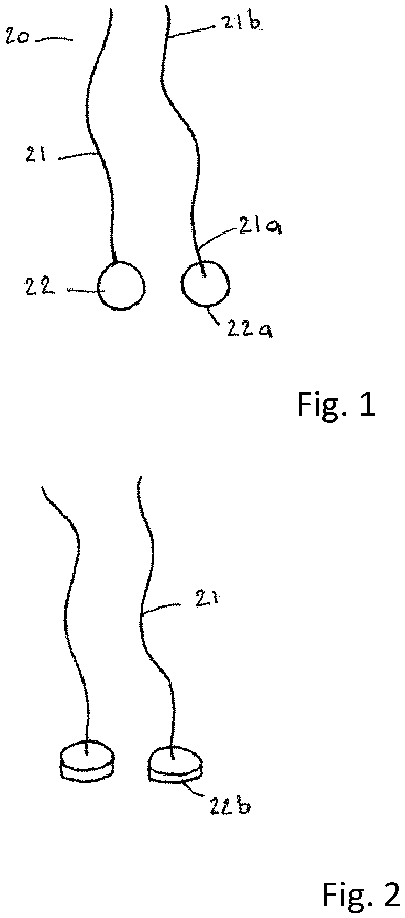

Les Figures 1 à 4 sont des vues en perspective d'un moyen de suspension 20, avec plusieurs modes de réalisation selon l'invention. Le moyen de suspension 20 comprend une paire de fils 21 avec une extrémité inférieure 21a et une extrémité supérieure 21b. L'extrémité inférieure 21a de chacun des fils 21 comprend au moins un élément suspenseur 22 fixé solidement à chaque extrémité inférieure 21a. L'extrémité supérieure 21b de chaque fil 21 de la paire de fils 21 est libre. La paire de fils 21 est composée d'un matériau biocompatible, non biodégradable avec une structure mono-filament. Le matériau composant le fil 21 peut être non métallique tel que du polypropylène, du polyvinylidenedifluoride (PVDF), ou tout autre matériau plastique synthétique biocompatible. Le matériau composant le fil 21 peut être de préférence métallique pour assurer une meilleure stabilité mécanique dans le temps et garantir l'absence d'étirement ainsi qu'une meilleure stabilité biologique dans le temps comparativement au matériau plastique synthétique. Parmi les métaux biocompatibles le titanium peut être utilisé entre autres. La structure mono-filament des fils 21 est utilisé de préférence au fil tressé de façon à avoir une surface lisse et prévenir la colonisation des fils 21 par les cellules des tissus traversés par les fils 21. La structure mono-filament facilite le glissement des tissus le long des fils 21 lors des efforts de toux, rire, éternuement. De même le fil 21 glisse facilement à travers les tissus traversés au moment du retrait du fil 21, au cas où le retrait du fil 21 serait nécessaire.

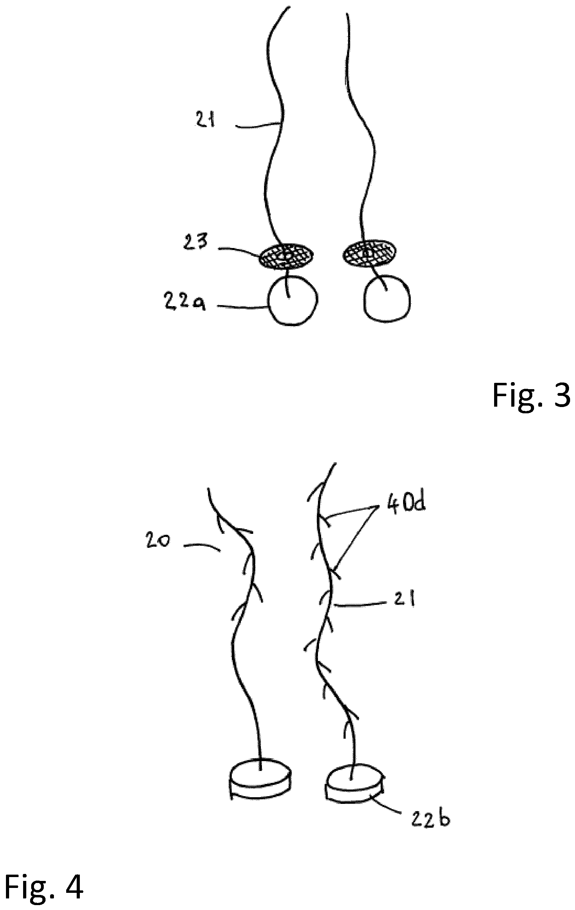

Comme montré dans la figure 4, le fil 21 peut présenter dans un mode de réalisation de l'invention des irrégularités en surface sous la forme de crans ou ardillons 40d présents sur toute la longueur ou seulement sur une partie de la longueur, préférentiellement sur la partie proche de l'extrémité supérieure 21b du fil 21. En dehors de cette caractéristique, les autres caractéristiques de ce type de fil 21 avec crans 40d sont identiques à celles décrites plus haut pour le fil 21 non cranté. La différence fonctionnelle entre ces deux types de fils 21 est que le glissement du fil 21 avec crans 40d ne se fait facilement que dans un seul sens au lieu des deux sens pour le fil 21 non cranté. Selon la figure 4 le glissement du fil 21 avec crans 40d se fait facilement dans la direction de l'extrémité supérieure 21b du fil 21. En d'autres termes, le glissement se fera facilement dans le sens opposé à celui de l'orientation des crans 40d orientés vers l'extrémité inférieure 21a du fil 21.

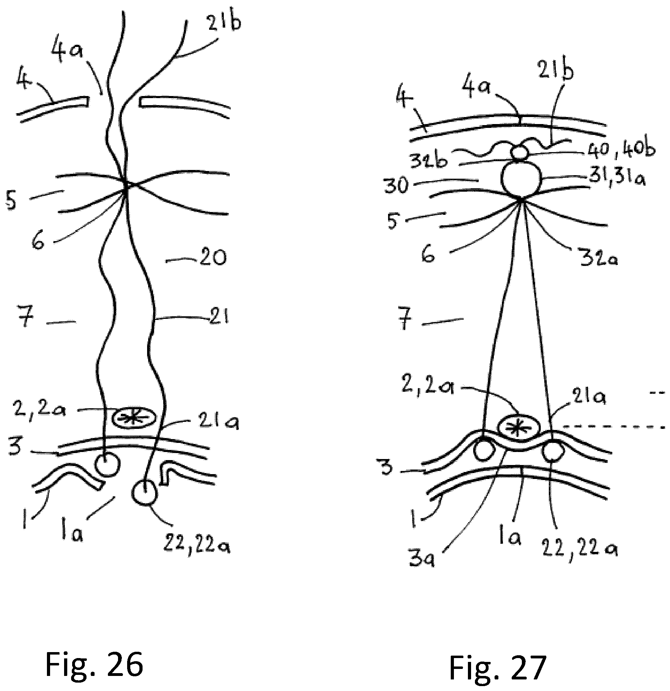

Comme montré dans les figures 1 à 4, l'élément suspenseur 22 peut se présenter sous la forme par exemple, d'une bille 22a, d'une plaque 22b, entres autres formes. La forme et le volume de l'élément suspenseur 22 ont pour but de prévenir la migration du fil 21 à travers les tissus, préférentiellement le plan musculo-aponévrotique pelvien 3, en direction de l'extrémité supérieure 21b du fil 21 et de permettre leur repérage par palpation digitale ou par échographie à travers la paroi du vagin 1 (voir ci-dessous la description des figures 26 à 28).

Comme montré dans la figure 3, une plaque 23 perforée en son centre peut être mise en place au contact de l'élément suspenseur 22. La plaque 23 est grillagée ou tissée. Les pores entre les mailles de la plaque 23 sont colonisés par les tissus. La plaque 23 augmente la résistance des tissus et renforce la prévention de la migration de l'élément suspenseur 22 à travers les tissus en contact avec ledit élément suspenseur 22. Le matériau composant la plaque est biocompatible, non résorbable, soit non métallique comme le polypropylène ou le polyvinylidénedifluoride (PVDF) ou bien métallique comme le titanium entre autres.

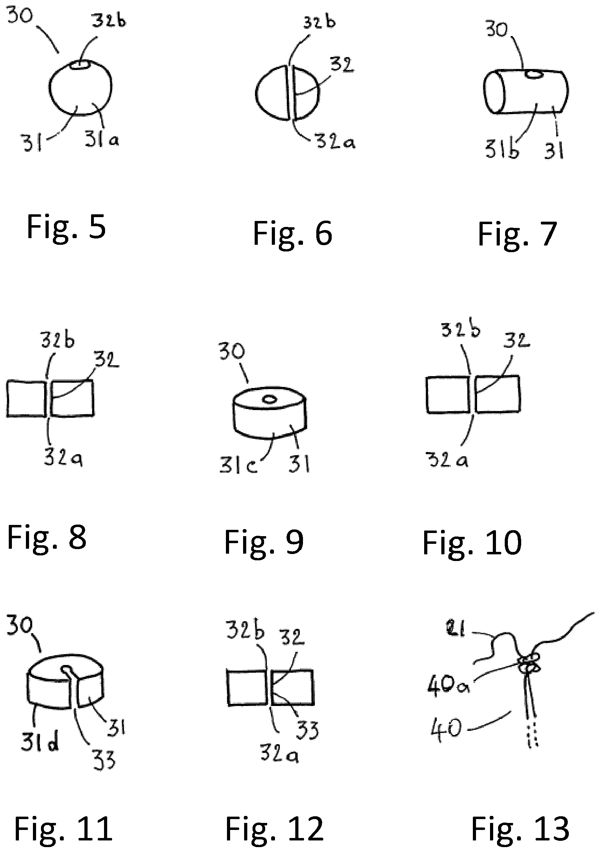

Les figures 5 à 12 sont des représentations en perspective et en coupe frontale d'un moyen de mise en tension 30 comprenant un élément de passage et d'appui 31. Les figures 13 à 20 sont des représentations en perspective et en coupe transversale d'un élément de blocage.

L'élément de passage et d'appui 31 peut avoir la forme d'une bille perforée 31a sur la figure 5, ou d'un cylindre perforé sur la figure 7 ou encore un disque perforé sur la figure 9. Chaque élément de passage et d'appui 31 présente un chenal 32 avec un orifice d'entrée 32a et un orifice de sortie 32b. Le chenal 32 passe au centre de la bille perforée 31a, au centre du cylindre 31b selon un axe perpendiculaire à son axe longitudinal, au centre d'un disque 31c. Le chenal 32 a un diamètre légèrement supérieur au diamètre des fils 21 du moyen de suspension 20. Selon la figure 11, le chenal 32 peut se prolonger par une fente radiale 33 de même hauteur que celle du chenal 32 et communiquant avec la périphérie du disque 31d. La largeur de la fente 33 est égale au diamètre du fil 21.

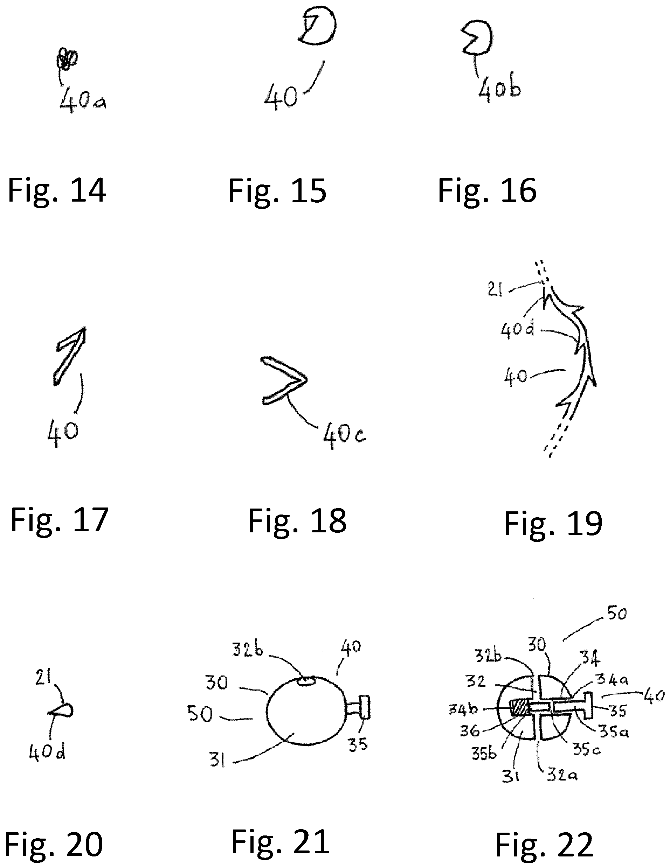

L'élément de blocage 40 représenté en perspective sur la figure 13 peut être l'empilement de nœuds 40a entre les deux fils 21 du moyen de suspension 20, sur la figure 15 un clip sphérique 40b avec une fente radiale allant du centre à l'extérieur du clip sphérique 40b, et sur la figure 17 un clip allongé 40c.

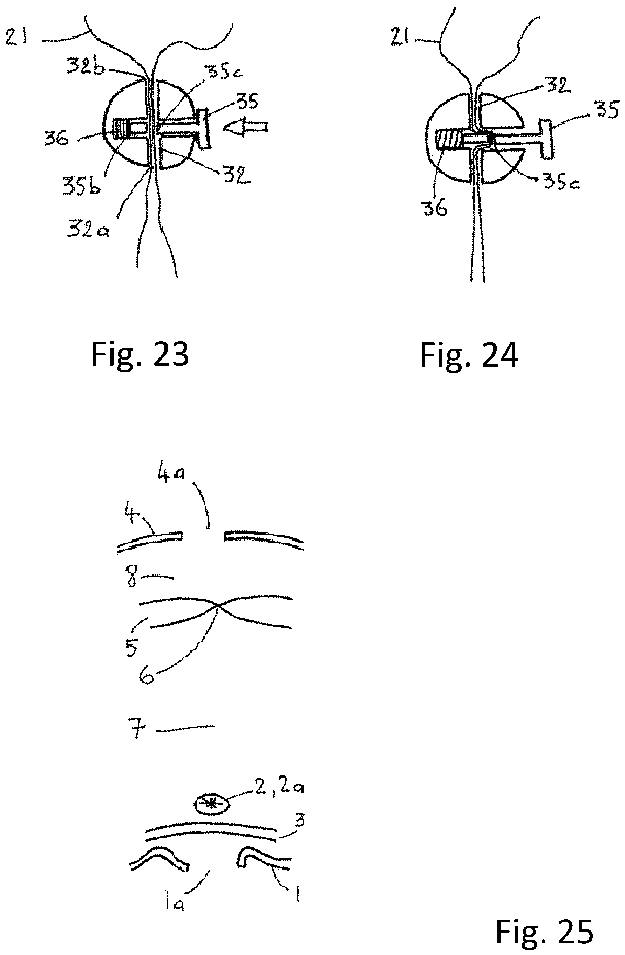

Dans une autre mode de réalisation représentée sur les figures 19 et 20, l'élément de blocage 40 est incorporé au fil 21 et se présente sous la forme de crans ou ardillons 40d sur toute la longueur du fil 21 ou préférentiellement sur une partie de l'extrémité supérieure 21b du fil 21 du moyen de suspension 20. Ce fil 21 avec crans 40d a comme caractéristique de pouvoir se bloquer lors de sa mobilisation dans les tissus dans la direction de la pointe des crans 40d et à l'opposé de pouvoir glisser lors de sa mobilisation dans les tissus dans le sens opposé, c'est-à-dire dans la direction de la base des crans 40d. La mobilisation du fil 21 en direction de la pointe des crans 40d déploie les crans 40d et bloque ainsi la mobilisation du fil 21. A l'opposé, la mobilisation du fil 21 en direction de la base des crans 40d aplatit les crans et permet le glissement du fil dans ladite direction. Cette caractéristique du fil 21 selon ce mode de réalisation est particulièrement intéressante car elle simplifie l'industrialisation du moyen de mise en tension 30 des fils 21. L'intérêt de ce type de fil 21 avec crans 40d sera précisé plus loin dans la description de la méthode d'utilisation du moyen de suspension 20 en association avec le moyen de mise en tension 30 des fils 21 dans le cadre du traitement de l'incontinence urinaire chez la femme (voir ci-dessous la description des figures 25 à 28).

Les éléments de blocage 40 sont composés de matériau biocompatible, non résorbable, déformable, métallique ou non métallique. Parmi les matériaux métalliques le titanium entre autres peut être utilisé, parmi les matériaux non-métalliques le plastique non résorbable des clips autobloquants pour clampage des vaisseaux peut être utilisé.

Les figures 21 à 24 représentent un moyen de mise en tension 30 dans lequel l'élément de blocage 40 est intégré à l'élément de passage et d'appui 30.

La figure 21 est une représentation en perspective d'une combinaison 50 entre l'élément de passage et d'appui 31 avec l'élément de blocage 40. L'orifice supérieure 32b ou de sortie du chenal 32 de l'élément de passage et d'appui 31 est visible, de même que le piston 35 de l'élément de blocage 40. La figure 22 est une coupe frontale de la combinaison 50. L'élément de passage et d'appui 31 est une bille 31a percée d'un chenal vertical 32 avec un orifice d'entrée inférieure 32a et un orifice de sortie supérieure 32b diamétralement opposés. Un deuxième chenal 34 horizontal est perpendiculaire au premier chenal 32 vertical. Le chenal 34 horizontal possède un orifice d'entrée 34a et un fond 34b. Ce chenal horizontal 34 est occupé par l'élément de blocage 40 sous la forme de l'axe 35a d'un piston 35. L'axe 35a du piston 35 présente une extrémité distale 35b. L'axe 35a du piston 35 est perforé par un chenal 35c perpendiculaire à l'axe 35a longitudinal du piston 35. Ce chenal 35c vertical peut être mis en alignement avec le chenal 32 vertical de la bille 31a. L'extrémité distale 35b du piston 35 est au contact d'un ressort 36 en appui sur l'extrémité borgne 34b du chenal horizontal 34. Selon la figure 23, l'alignement du chenal 35c et du chenal 32 s'observe, lorsque le piston 35 comprime complètement le ressort 36 en butée contre l'extrémité borgne 34 b de la bille 31a. Les deux fils 21 sont glissés par l'orifice d'entrée 32a dans le chenal vertical 32 de la bille perforée 31a puis dans le chenal vertical 35c du piston 35 et sortent par l'orifice de sortie 32b du chenal 32 de la bille 31a. Lorsque la pression sur le piston 35 est relâchée, le piston recule sous l'effet du ressort 36 et les fils 21 du moyen de suspension 20 prennent un trajet avec 4 angles droits comme représentés sur la figure 24. Les fils 21 sont ainsi bloqués dans la position souhaitée à l'intérieur de l'élément de passage et d'appui 31 des fils 21.

Les Figures 25 à 28 sont une représentation schématique d'une méthode d'utilisation d'un dispositif médical implantable selon les différents aspects de la présente invention.

Dans une première étape représentée par la figure 25, une première incision la est réalisée au niveau du vagin 1 d'un patient de sexe féminin. La première incision vaginale la est réalisée sur la ligne médiane du patient en regard de l'urètre 2 et sur une longueur d'environ 15 mm. Un passage latéral est créé de chaque côté de l'urètre par dissection avec une paire de ciseaux jusqu'au contact avec le plan musculo-aponévrotique pelvien 3. Une deuxième incision sus-pubienne 4a est ensuite réalisée au niveau de la peau de la région sus-pubienne 4 sur la ligne médiane. Un passage est réalisé par dissection dans la graisse sus-pubienne 8 jusqu'au contact avec le plan musculo-aponévrotique des muscles grands droits 5 de la paroi abdominale sur la ligne médiane sus-pubienne 6. L'incision sus-pubienne 4a est réalisée sur une longueur de 20 mm environ. La longueur peut être légèrement plus importante et adaptée à l'épaisseur de la graisse sus-pubienne 8.

Dans une deuxième étape représentée par la figure 26 un moyen de suspension 20 est mis en place. Une paire de fils 21 avec chacun un élément suspenseur 22, une bille 22a par exemple, est insérée à partir de l'incision vaginale la. L'extrémité supérieure 21b d'un fil 21 est insérée de chaque côté de l'urètre 2, puis glissée dans la graisse de l'espace rétro-pubien 7, puis passée à travers le plan musculo-aponévrotique des muscles grands droits 5 sur la ligne médiane sus-pubienne 6, pour sortir par l'incision sus-pubienne 4a. A ce stade, les fils 21 sont dans un état relâché : en effet, la bille 22a au niveau de l'extrémité inférieure 21a de chacun des deux fils 21 n'est pas en contact avec le plan musculo-aponévrotique pelvien 3 et l'extrémité supérieure 21b de chacun des deux fils 21 est libre au niveau de l'incision sus-pubienne 4a.

Dans une troisième étape représentée par la figure 27 un moyen de mise en tension est mis en place. Dans un premier temps un moyen de passage et d'appui 31 est mis en place. L'extrémité supérieure 21b libre de chacun des fils 21 est glissée à travers l'orifice inférieur 32a du chenal 32 (non représenté) de la bille perforée 31a et sort par son orifice supérieur 32b. Dans un deuxième temps, une traction est exercée au niveau de l'extrémité supérieure 21b de la paire de fils 21, jusqu'à ce que la bille 22a à l'extrémité inférieure 21a de chacun des fils 21 soit en contact avec le plan musculo-aponévrotique pelvien 3. Une bille 22a est placée de chaque côté de l'urètre 2. L'urètre 2 repose alors sur un hamac 3a naturel musculo-aponévrotique suspendu par et entre les deux billes 22a, une bille 22a de chaque côté de l'urètre 2. Dans un troisième temps la position et la tension des fils 21 est bloquée par la mise en place d'un élément de blocage 40, par exemple un clip sphérique 40b. Dans le cas d'une paire de fils 21 avec crans 40d comme décrit plus haut par les figures 19 et 20, la position et la tension des fils 21 sont bloquées automatiquement dès le contact des billes 22a avec le plan musculo-aponévrotique pelvien 3. Les crans 40d prennent appui sur l'élément de passage et d'appui 31 du moyen de mise en tension 30 du dispositif. Les crans 40d s'écartent sous l'effet de la tension des fils 21. La position et la tension des fils 21 avec crans 40d est automatiquement bloquée. Une fois le blocage des fils 21 réalisés, l'extrémité supérieure 21b de chacun des fils 21 est coupée en laissant dépasser au-delà de l'élément de blocage 40 une longueur de fil d'environ 3 cm. Cette partie du fil 21 est lâche et se trouve dans la graisse 8 de la région sus-pubienne. L'incision sus-pubienne 4a est alors fermée, de même que l'incision vaginale la.

Au moment d'une augmentation de la pression abdominale sous l'effet d'un effort, de toux, de rire, d'éternuement, la pression est transmise intégralement au niveau de tous les organes de la cavité abdominale dont la vessie et l'urètre 2. La vessie et l'urètre 2 sont mobilisés vers le bas. Lorsque le tonus du sphincter urétral 2a est insuffisant, une fuite d'urine apparait. Après mise en place d'un dispositif comme décrit plus haut, toute augmentation de pression abdominale est transmise à l'urètre 2. La position du hamac 3a musculo-aponévrotique pelvien étant fixe, l'urètre 2 bute contre le hamac 3a et se trouve comprimé contre ledit hamac 3a. Plus la pression abdominale est forte, plus la compression de l'urètre 2 est forte. La continence urinaire est assurée. La miction est possible par le simple relâchement de la pression abdominale et par le relâchement réflexe du tonus du sphincter urétral 2a.

Dans une mode de réalisation de la technique décrite ci-dessus chaque fil 21 de la paire de fils 21 est mis en tension et bloqué séparément. Le montage nécessite la mise en place pour chaque fil 21 d'un moyen de mise en tension 30 séparé.

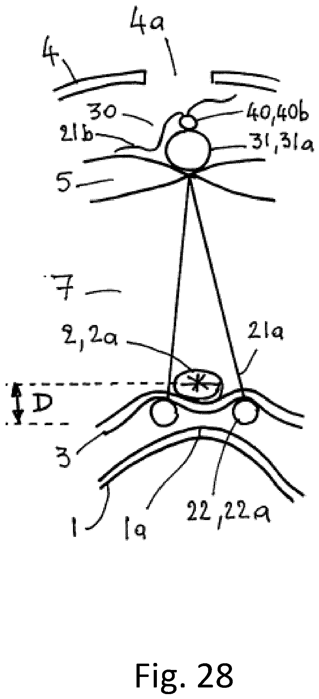

La figure 28 est une représentation schématique de la technique de réajustement de la tension des fils 21, dans le cas où à distance de la mise en place du dispositif selon les figures 25 à 27, les fuites urinaires réapparaissent après une période de guérison temporaire.

Après repérage par palpation digitale ou instrumentale de l'élément de passage et d'appui 31 par exemple une bille 31a, une incision est réalisée en regard de la bille 31a. La bille 31a, l'élément de blocage 40, par exemple un clip sphérique 40b, et les fils 21 sont exposés. La traction sur les fils 21 mobilise les fils monobrin lisse qui glissent dans la graisse 7 de l'espace rétro-pubien et à travers le plan musculo-aponévrotique des muscles grands droits 5 de l'abdomen. Cette traction permet de réajuster la position et la tension de la paire de fils 21. Dans leur nouvelle position et leur nouvelle tension, les fils 21 sont bloqués par un deuxième clip sphérique positionné entre l'élément de passage et d'appui 31 par exemple la bille perforée 31a et le premier clip sphérique 40b. Le premier clip sphérique 40b est retiré ou laissé en place. L'incision sus-pubienne 4a est fermée. La position du plancher musculo-aponévrotique pelvien 3 et de l'urètre 2 a été relevée d'une distance D par rapport à leurs positions respectives immédiatement après la mise en place initiale du dispositif.

Dans le cas où la miction est difficile avec un résidu vésical post-mictionnel significatif, le réajustement avec relâchement de la tension des fils 21 est également possible. Ce cas est moins fréquent que le réajustement avec augmentation de la tension des fils. Après retrait de l'élément de blocage 40 des fils 21, une traction exercée vers le bas sur l'urètre 2 avec une bougie permet d'abaisser l'urètre et de relâcher les fils 21. Les fils 21 sont alors bloqués dans leur nouvelle position et nouvelle tension par la mise en place d'un nouvel élément de blocage 40. Dans le cas de fils 21 avec crans 40d une simple traction vers le bas exercée à l'aide d'une bougie introduite à l'intérieur de l'urètre 2 permet de faire glisser les crans 40d des fils 21 le long du chenal 32 de l'élément de passage et d'appui 31. Les fils se bloquent automatiquement dans leur nouvelle position et nouvelle tension par les crans 40d prenant appui contre l'orifice supérieur 32b et à l'intérieur du chenal 32 de l'élément de passage et d'appui 31.

Dans le cas où une complication nécessite le retrait partiel ou complet du dispositif, la méthode et technique de retrait est la suivante.

Après repérage par palpation digitale ou instrumentale et incision de la paroi du vagin 1 en regard de l'élément suspenseur 22, la bille 22a et le fil 21 dans sa version non crantée sont exposés. Après repérage par palpation digitale ou instrumentale et incision de la peau dans la région sus-pubienne 4 en regard de l'élément de passage et d'appui 31, l'élément de passage et d'appui 31 ainsi que les extrémités supérieures des fils 21b bloqués par l'élément de blocage 40 sont exposés.

Dans le cas d'une paire de fils 21 non crantés, le retrait du dispositif peut se faire par section au niveau de l'incision vaginale de l'extrémité inférieure 21a du fil 21 au-dessus de l'élément suspenseur 22. L'élément suspenseur 22 est retiré par le vagin 1 et le fil 21 est retiré par la région sus-pubienne en bloc avec l'élément de passage et d'appui 31 ainsi que l'élément de blocage 40. Le retrait du dispositif peut également se faire après section des extrémités supérieures 21b des fils en dessous de l'élément de passage et d'appui 31. Ce dernier ainsi que l'élément de blocage 40 sont retirés par l'incision cutanée sus-pubienne 4a, alors que les éléments de suspension 22 et les fils 21 sont retirés par traction à partir de l'incision vaginale la.

Dans le cas de l'application du dispositif à la prévention de l'incontinence urinaire secondaire au traitement chirurgical selon la technique de prostatectomie radicale pour cancer de la prostate chez l'homme, le dispositif sera mis en place préventivement chez un patient jugé à haut risque d'incontinence du fait d'un cancer de prostate localement avancé et éventuellement une pression de clôture du sphincter strié de l'urètre diminuée. Après réalisation de l'anastomose vésico-urétrale, un élément suspenseur 22 est placé de chaque côté de l'anastomose, la paire de fils 21 sort dans la région sus-pubienne, passe à travers l'élément de passage et d'appui 31. Les fils ne sont pas mis en tension, ils sont simplement mis en attente d'une éventuelle mise en tension. L'extrémité libres 21b des fils sont abandonnées dans la graisse sus-pubienne 8 et l'incision sus-pubienne 4a est fermée. Lorsque, plus de 6 mois après l'opération, le patient présente toujours des fuites urinaires significatives, la mise en tension des fils est réalisée. Après repérage au niveau de la peau de la région sus-pubienne 4 par palpation digitale ou instrumentale de l'élément de passage et d'appui 31, une incision est réalisée en regard de l'élément de passage et d'appui 31. Ce dernier est libéré de même que les fils 21. La procédure de mise en tension et de blocage des fils est alors identique à celle décrite chez la femme selon la représentation schématique et la description détaillée de la figure 28. Lorsqu'il n'y a pas de fuites urinaires après l'opération le matériel implanté est laissé en place définitivement.

Alors que l'invention est susceptible de modifications des modes de réalisations, certains aspects spécifiques sont décrits en détail à l'aide des figures ci-dessous, sans que ces aspects représentent une limitation à l'invention. En effet l'invention entend couvrir toutes modifications, équivalents, et alternatives tombant dans le domaine de l'invention comme défini par les revendications attenantes.

### Légendes des figures

### Anatomie

- 1: vagin ou paroi vaginale
- 1a: incision vaginale
- 2: urètre
- 2a: sphincter urétral
- 3: plan musculo-aponévrotique pelvien
- 3a: hamac
- 4: peau sus-pubienne, région sus-pubienne
- 4a: incision de la région sus-pubienne
- 5: muscle grand droit de l'abdomen
- 6: ligne blanche de la région sus-pubienne
- 7: espace rétro-pubien ou graisse rétro-pubienne
- 8: tissu graisseux sous-cutané de la région sus-pubienne

### Moyen de suspension :

- 20: moyen de suspension
- 21: fil de suspension
- 21a: extrémité proximale
- 21b: extrémité distale
- 22: élément de suspension
- 22a: bille
- 22b: bouton ou plaque circulaire
- 23: rondelle perforée tressée ou grillagée
- 24: cran ou ergot

Moyen de mise en tension : combinaison de deux éléments
- élément de passage, d'appui et de repérage
- élément de blocage

### Elément de passage, d'appui et de repérage

- 30: moyen de mise en tension
- 31: élément de passage et d'appui
- 31a: bille ou sphère perforée
- 31b: cylindre perforé
- 31c: disque perforé
- 33: fente (communiquant avec le chenal et perpendiculaire à l'axe du disque)
- 32: chenal vertical
- 32a: orifice d'entrée
- 32b: orifice de sortie

### Elément de blocage

- 40: élément de blocage
- 40a: nœud
- 40b: clip sphérique fendu
- 40c: clip allongé
- 40d: cran ou ardillon

### Intégration de l'élément de blocage à l'élément de passage, d'appui et de repérage

- 50: unité de mise en tension
- 31: bille
- 32: chenal
- 32a: orifice d'entrée
- 32b: orifice de sortie

- 34: chenal horizontal
- 34a: orifice d'entrée
- 34b: fond du chenal
- 35: piston
- 35a: axe du piston
- 35b: extrémité distale du piston
- 35c: chenal dans l'axe 35a du piston 35
- 36: ressort

## Revendications

1. Dispositif médical pour le traitement de l'incontinence urinaire comprenant :
- au moins un fil de suspension (21) présentant une première extrémité comportant un élément de suspension et une seconde extrémité libre,
- au moins un moyen de mise en tension (30) d'un fil de suspension comprenant un élément de passage et d'appui (31) pour la seconde extrémité dudit fil de suspension pour un élément de blocage (40) de la tension du fil de suspension, ledit élément de passage et d'appui présentant au moins un chenal (32) avec un orifice d'entrée (32a) et un orifice de sortie (32b), et ledit élément de blocage de la tension du fil de suspension étant conçu pour bloquer de manière amovible et réversible la seconde extrémité du fil de suspension au-delà de l'orifice de sortie dudit élément de passage et d'appui.

2. Dispositif selon la revendication 1, dans lequel le fil de suspension (21) est biocompatible, monobrin, non résorbable, mobilisable, amovible, non métallique ou métallique, de surface lisse ou avec crans ou ardillons disposés le long de la surface du fil et dans lequel l'élément de suspension à l'extrémité opposée à l'extrémité libre dudit fil est une bille (22a), un disque de surface lisse en matériau biocompatible métallique ou non métallique tel que titanium, verre, céramique, polypropylène ou autre composant synthétique

3. Dispositif selon la revendication 1, dans lequel l'élément de passage (31) du moyen de mise en tension (30) est biocompatible, non résorbable, mobilisable, amovible, métallique ou non métallique, est une bille (31a), un cylindre (31b) ou un disque perforé(e) (31c) avec un chenal (32) dont le diamètre est adapté au diamètre du fil (21) et du moyen de blocage, de façon à garantir à l'élément de blocage (40) du moyen de mise en tension un appui ou accroche efficace sur l'élément de passage.

4. Dispositif selon la revendication 1, dans lequel l'élément de blocage (40) du moyen de mise en tension (30) est biocompatible, non résorbable, mobilisable, métallique ou non métallique, amovible et repositionnable.
